# EUROPEAN PATENT APPLICATION

(11) **EP 2 636 395 A1**
(43) Date of publication of application: **11.09.2013**
(21) Application number: 13170964.4
(22) Date of filing: 20.08.2008
(51) Int. Cl.: A61F 13/15, A61F 13/472, A61F 13/53, A61F 13/539, A61F 13/531

(54) **Absorbent body**

(30) Priority: 24.08.2007 JP 2007219023; 24.08.2007 JP 2007219024
(62) Divisional of application: 08828066.4
(71) Applicant: Uni-Charm Corporation, Ehime 799-0111 (JP)
(72) Inventor: Kudo, Jun, Kagawa, 769-1602 (JP); Kinoshita, Hideyuki, Kagawa, 769-1602 (JP); Hashino, Akira, Kagawa, 769-1602 (JP)
(74) Representative: Peter, Julian

(57) **Abstract**

The present invention relates to an absorbent body, having a liquid-absorbent core, and a sheet that wraps the liquid-absorbent core, and that has a skin-contacting face positioned on a side where the sheet contacts a user's body, and a non-skin-contacting face positioned on a side where the sheet does not contact the user's body, the absorbent body having a width direction and a longitudinal direction intersecting the width direction, the absorbent body having a joined section where a part of the sheet on the non-skin-contacting face and another part of the sheet on the non-skin-contacting face are joined in such a manner as the part and the other part oppose on a center in the width direction along the longitudinal direction.

## Description

### Technical Field

The invention relates to absorbent articles for absorbing fluid and absorbent bodies.

### Background Art

Absorbent articles have been conventionally known that include an absorbent body for absorbing a certain fluid such as menstrual blood. Some of the absorbent articles have, for example, a two-layer structure including a first absorbent body (base body) and a second absorbent body (absorbent body) that is placed on the first absorbent body. In addition, an absorbent article has been proposed in which the second absorbent body separates from the first absorbent body and is used with being sandwiched between a recess such as buttocks of user's body in order to cause the second absorbent body to be in close contact with the recess. (See JP-A-2002-159534, for example).

Further, absorbent articles have conventionally been known that include an absorbent body for absorbing a certain fluid such as menstrual blood. Some of the absorbent articles have a two-layer structure including a first absorbent body and a second absorbent body that is placed on the first absorbent body. An absorbent article has been proposed whose second absorbent body, for example, is shaped having a trefoil-shaped cross-section in order to fit user's body. (See JP-T-2002-512851, for example).

### Disclosure of Invention

### Problem to be Solved by the Invention

However, in the absorbent article described in JP-A-2002-159534, the second absorbent body is sandwiched between the recess with separating from the first absorbent body. Therefore, there is a risk that the second absorbent body is removed from the recess when user's movement is large.

The Invention has been made in view of conventional problems as described above, and an advantage thereof is to provide an absorbent article that is likely to be in close contact with user's body.

Further, regarding the second absorbent body of the absorbent article described in JP-T-2002-512851, its manufacturing method is complex; that is, the second absorbent body is shaped having a trefoil-shaped cross-section by being folded in three in such a manner as end sections of one face (skin-contacting face) of a sheet-like composite web containing absorbent materials oppose.

The Invention has been made in view of conventional problems as described above, and an advantage thereof is to provide an absorbent body that is likely to be in close contact with user's body and whose manufacturing method is simple, and an absorbent article.

### Means for Solving the Problem

In order to solve the above-described problems, a principal aspect of the invention is an absorbent article, including: a base body that includes a fluid-permeable surface sheet, a fluid-impermeable back face sheet, and a liquid-absorbent core between the surface sheet and the back face sheet, and that has a width direction and a longitudinal direction intersecting the width direction; and, that has, at both side sections in the width direction, compressed sections in which the fluid-permeable surface sheet and the liquid-absorbent core are compressed along the longitudinal direction of the base body in either one of a continuous manner and an discontinuous manner; and an absorbent body that includes a liquid-absorbent member, that has a width direction and a longitudinal direction intersecting the width direction, and that has a minimum section whose length along the width direction of the absorbent body is shortest; the absorbent body being place on the fluid-permeable surface sheet of the base body in such a manner as the longitudinal direction of the base body is the same as the longitudinal direction of the absorbent body, in a portion that is in the base body and on which the minimum section of the absorbent body is placed, a length between a pair of the compressed sections provided in the both side sections, along the width direction of the base body, being equal to or more than a length of the minimum section of the absorbent body along the width direction of the absorbent body.

Further, in order to solve the above-described problems, a principal aspect of the invention is an absorbent body, including: a liquid-absorbent core; and a sheet that wraps the liquid-absorbent core, and that has a skin-contacting face positioned on a side where the sheet contacts a user's body, and a non-skin-contacting face positioned on a side where the sheet does not contact the user's body; the absorbent body having a width direction and a longitudinal direction intersecting the width direction, the absorbent body having a joined section where a part of the sheet on the non-skin-contacting face and another part of the sheet on the non-skin-contacting face are joined in such a manner as the part and the other part oppose on a center in the width direction along the longitudinal direction.

### Effects of the Invention

With the invention, it is possible to provide an absorbent article that can easily be in close contact with user's body. Besides, with the invention, it is possible to provide an absorbent body that is likely to be in close contact with user's body and whose manufacturing method is simple, and an absorbent article.

### Brief Description of Drawings

[FIG. 1] This is a plan view of a surface side of a base absorbent body.
[FIG. 2] This is a plan view of a surface side of a top absorbent body.
[FIG. 3] This is a diagram showing a state in which the base absorbent body and the top absorbent body are joined.
[FIG. 4] This is a perspective view of a sanitary napkin 1.
[FIG. 5] FIGS. 5A to 5C are cross-sectional views of the base absorbent body.
[FIG. 6] FIGS. 6A to 6E are cross-sectional views of the top absorbent body.
[FIG. 7] This is a diagram showing the distribution of basis weights of pulverized pulp in the top absorbent body.
[FIG. 8] FIG. 8A is a cross-sectional view before a narrow region is formed, FIG. 8B is a cross-sectional view after the narrow region is formed, and FIG. 8C is a diagram how the narrow region abuts a recess of user's body.
[FIG. 9] FIG. 9A is a cross-sectional view of a sanitary napkin according to the present embodiment, and FIG. 9B is a cross-sectional view of a sanitary napkin according to a comparative example.
[FIG. 10] FIG. 10A is a diagram showing how the sanitary napkin is wrapped individually, and FIG. 10B is a diagram showing a modified example of individual wrapping.
[FIG. 11] FIG. 11A is a plan view and a cross-sectional view of a top absorbent body according to the comparative example, and FIG. 11B is a diagram showing how the top absorbent body according to the comparative example abuts a recess of user's body.
[FIG. 12] This is a diagram showing how a top absorbent body according to the comparative example abuts a recess of user's body.
[FIG. 13] This is a plan view of the surface side of the base absorbent body.

### List of Reference Numerals

1 sanitary napkin (absorbent article)
10 base absorbent body (base body), 10a front end section, 10b rear end section, 10g permanently-joined section 10g, 12 absorbent-body base material, 12a pulverized pulp layered body (liquid-absorbent core), 14 surface sheet (fluid-permeable surface sheet), 15 compressed recess (compressed section), 16 temporarily joined section, 17 curved section, 20 top absorbent body (absorbent body, liquid-absorbent core), 20a front end section, 20b rear end section, 22 pulverized pulp layered body (liquid-absorbent member), 23 intermediate sheet, 24 shape-keeping sheet (sheet), 25 sealed section, 26 sealed section, 27 fastening section, 28 perforation,
30 back face sheet (fluid-impermeable back face sheet), 30a front end section, 30b rear end section,
32 wing section, 33 anti-displacement affixing section, 34 protection sheet 34,
35 wrapping sheet, 36 tape, 40 side sheet, 44 fixed section, 46 end section, 48 elastic member, 50 leakage-proof sheet, 51 narrow region, 52 joined section, 53 first region, 54 second region, 90 undergarment, 91 recess,
CL center line, Z point that is supposed to face vaginal orifice

### Best Mode for Carrying Out the Invention

At least the following matters will be disclosed in the description in the present specification and the accompanying drawings.

An absorbent article, including: a base body that includes a fluid-permeable surface sheet, a fluid-impermeable back face sheet, and a liquid-absorbent core between the surface sheet and the back face sheet, and that has a width direction and a longitudinal direction intersecting the width direction; and, that has, at both side sections in the width direction, compressed sections in which the fluid-permeable surface sheet and the liquid-absorbent core are compressed along the longitudinal direction of the base body in either one of a continuous manner and an discontinuous manner; and an absorbent body that includes a liquid-absorbent member, that has a width direction and a longitudinal direction intersecting the width direction, and that has a minimum section whose length along the width direction of the absorbent body is shortest; the absorbent body being place on the fluid-permeable surface sheet of the base body in such a manner as the longitudinal direction of the base body is the same as the longitudinal direction of the absorbent body, in a portion that is in the base body and on which the minimum section of the absorbent body is placed, a length between a pair of the compressed sections provided in the both side sections, along the width direction of the base body, being equal to or more than a length of the minimum section of the absorbent body along the width direction of the absorbent body.

With this absorbent article, if the base body is sandwiched between user's thighs when being worn, a region between the compressed sections curves toward the body at the pair of compressed sections. Therefore, the absorbent body placed into user's recess can be supported by the region between the compressed sections. As the above-mentioned absorbent article, the length between the pair of compressed sections along the width direction is equal to or more than the length of the region of the absorbent body (minimum section) that abuts user's recess along the width direction. This makes it possible to support the absorbent body placed into the recess by the wide base body, and also makes it possible to keep, in a stable manner, the absorbent body placed into the recess and in close contact with user's body.

In such an absorbent article, the minimum section is provided in a middle part in the longitudinal direction of the absorbent body.

With this absorbent article, the minimum section of the absorbent body is likely to abut the recess of user's body.

In such an absorbent article, the middle part in the longitudinal direction of the absorbent body can separate from the base body.

With this absorbent article, the region of the absorbent body (the middle part in longitudinal direction) that abuts user's recess can separate from the base body. Therefore, when a user wears the absorbent article, it is easy to place the absorbent body into user's recess. When the absorbent body is placed into the recess, the region of the base body between the compressed sections curves toward user's body by being sandwiched between thighs. Accordingly, it is possible to keep in a stable manner the absorbent body placed into the recess of user's body.

In such an absorbent article, three or more of the compressed sections are provided in the portion that is in the base body and on which the minimum section of the absorbent body is placed, and the pair of compressed sections consists of, among the compressed sections provided in the portion on which the minimum section is placed, a compressed section that is located closest to one side in the width direction, and a compressed section that is located closest to another side in the width direction.

With this absorbent article, the base body is likely to curve at the compressed sections that are provided on the base body and that are respectively located closest to each of the both sides in the width direction. Therefore, if a length between the compressed sections respectively provided closest to each of the both sides along the width direction is equal to or more than a length of the minimum section of the absorbent body along the width direction, it is possible to keep in a stable manner the absorbent body placed into the recess.

In such an absorbent article, the absorbent body includes a sheet that wraps the liquid-absorbent member, and that has a skin-contacting face positioned on a side where the sheet contacts a user's body, and a non-skin-contacting face positioned on a side where the sheet does not contact the user's body, and the middle part in the longitudinal direction of the absorbent body is a narrow region in which a part of the sheet on the non-skin-contacting face and another part of the sheet on the non-skin-contacting face are joined in such a manner as to oppose each other on a center in the width direction along the longitudinal direction of the absorbent body.

With this absorbent article, a length of the middle part in the longitudinal direction of the absorbent body along the width direction can be less than lengths of other regions of the absorbent body along the width direction. This makes it easy to place the absorbent body into the user's recess. In addition, since all that is required is to join the parts of the sheet, a manufacturing method is simple.

In such an absorbent article, in a portion that is in the base body and on which the narrow region is placed, a length between the pair of compressed sections along the width direction of the base body is equal to or more than a length of the narrow region along the width direction of the absorbent body.

With this absorbent article, since the region of the absorbent body (narrow region) that abuts user's recess is supported by the wide base body, it is possible to keep in a stable manner the absorbent body placed into the recess.

In such an absorbent article, the narrow region is located between a first region and a second region in the longitudinal direction of the absorbent body, a length of the first region along the width direction of the absorbent body is longer than the length of the narrow region along the width direction, and a length of the second region along the width direction of the absorbent body is longer than the length of the narrow region along the width direction.

With this absorbent article, the regions other than the narrow region (first region and second region) that abuts the recess of user's body are wider than the narrow region. Therefore, an appearance of the absorbent article can give a user a sense of security. In addition, for example, when the first region abuts a vaginal orifice, fluid can be received with a wide region. Further, the first region and the second region have a comparatively flat shape. Therefore, for example, when the second region abuts buttocks (waist), it is possible to prevent a user from feeling uncomfortable when the user is lying.

In such an absorbent article, a weight of the narrow region is less than a weight of the first region and a weight of the second region.

With this absorbent body, the parts of the sheet are easy to be joined by a joined section. In addition, it is possible to reduce the consumption of the liquid-absorbent core.

An absorbent body, including: a liquid-absorbent core; and a sheet that wraps the liquid-absorbent core, and that has a skin-contacting face positioned on a side where the sheet contacts a user's body, and a non-skin-contacting face positioned on a side where the sheet does not contact the user's body; the absorbent body having a width direction and a longitudinal direction intersecting the width direction, the absorbent body having the joined section where a part of the sheet on the non-skin-contacting face and another part of the sheet on the non-skin-contacting face are joined in such a manner as the part and the other part oppose on a center in the width direction along the longitudinal direction.

With this absorbent body, a region in which the part and the other part of the sheet are joined is narrow in the width direction. Therefore, it is easy to place the absorbent body into the recess of the user's body (perineum), and also the absorbent body is likely to be in close contact with the user's body. Besides, since all that is required is to join the parts of the sheet, a manufacturing method is simple and an inexpensive mass-production is possible.

In such an absorbent body, the absorbent body has a vaginal-orifice opposing section that opposes a vaginal orifice of the user's body, one end of the absorbent body in the longitudinal direction is located on a side close to buttocks of the user's body, and the joined section is located between the vaginal-orifice opposing section and the one end, in the longitudinal direction.

With this absorbent body, since a region whose width in the width direction is shortened by the joined section opposes the recess of the user's body, the absorbent body is likely to be in close contact with the recess of the user's body.

In such an absorbent body, a narrow region whose length along the width direction is a certain width is located between a first region and a second region in the longitudinal direction, a length of the first region along the width direction is longer than the certain width, a length of the second region along the width direction is longer than the certain width, and the narrow region is a region in which the part and the other part are joined by the joined section.

With this absorbent body, the regions other than the narrow region (first region and second region) that abuts the recess of user's body are wider than the narrow region. Therefore, an appearance of the absorbent body can give a user a sense of security. In addition, for example, when the first region abuts a vaginal orifice, fluid can be received with a wide region. Further, the first region and the second region have a comparatively flat shape. Therefore, for example, when the second region abuts buttocks (waist), the absorbent body does not make a user feel uncomfortable when the user is lying.

In such an absorbent body, a weight of the narrow region is less than a weight of the first region and a weight of the second region.

With this absorbent body, the parts of the sheet are easy to be joined by the joined section. In addition, it is possible to reduce the consumption of the liquid-absorbent core.

In such an absorbent body, the joined section does not exist on a folding position that is along the width direction and where the absorbent body is folded.

With this absorbent body, even if the absorbent body is folded in a state in which the joined section joins the parts of the sheet in individual wrapping, the joined section is unlikely to split. Also, the absorbent body is easy to be folded.

Further, an absorbent article, including: a base body having a fluid-impermeable sheet; an absorbent body that is provided placed on the base body; that has a width direction and a longitudinal direction intersecting the width direction, and that has a liquid-absorbent core and a sheet wrapping the liquid-absorbent core, the sheet having a skin-contacting face positioned on a side where the sheet contacts a user's body, and a non-skin-contacting face positioned on a side where the sheet does not contact the user's body, the absorbent article having a joined section where a part of the sheet on the non-skin-contacting face and another part of the sheet on the non-skin-contacting face are joined in such a manner as the part and the other part oppose on a center in the width direction along the longitudinal direction.

With this absorbent article, it is possible to easily manufacture an absorbent article that is likely to be in close contact with a body.

### === Present Embodiments ===

### <Brief Configuration of Sanitary Napkin>

Hereinafter, an absorbent article is described as a sanitary napkin. Hereinafter, a side of the sanitary napkin that contacts user's body is referred to as a surface side, and a side that contacts an undergarment is referred to as a back face side. The sanitary napkin is long in a certain direction as a whole. This certain direction is defined as a longitudinal direction, and a direction intersecting the longitudinal direction is defined as a width direction. A direction normal to a surface or back face of the sanitary napkin is defined as a thickness direction. First, the brief configuration of the sanitary napkin is described.

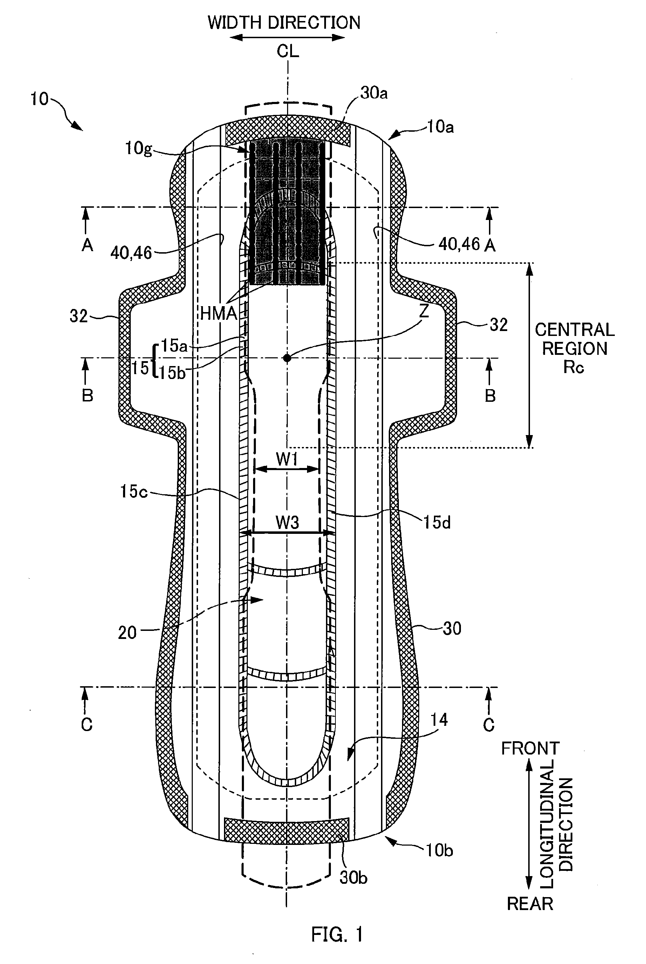

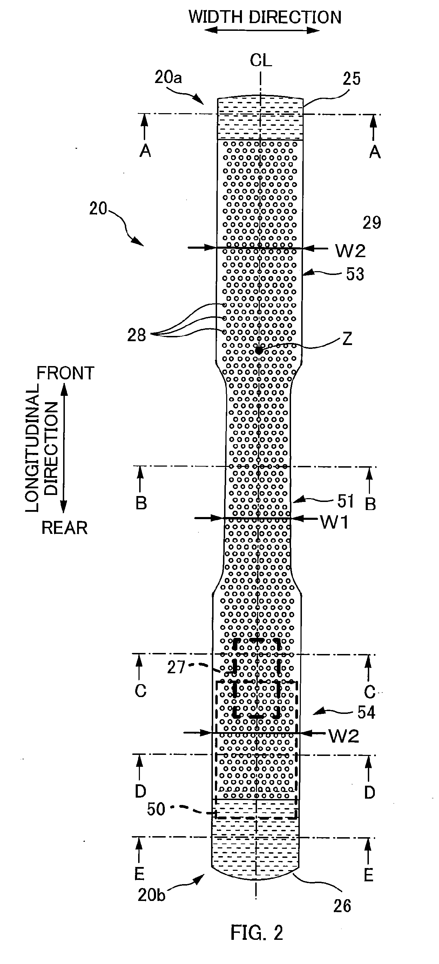

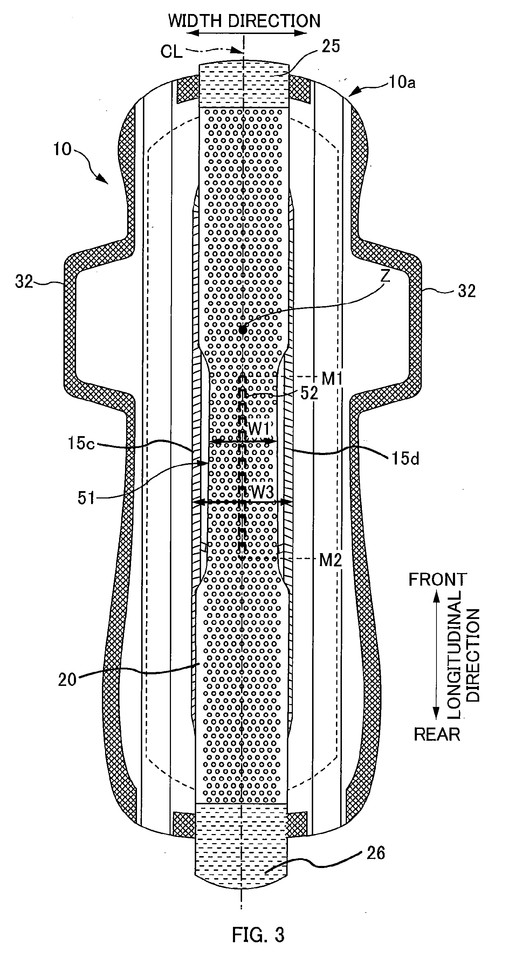

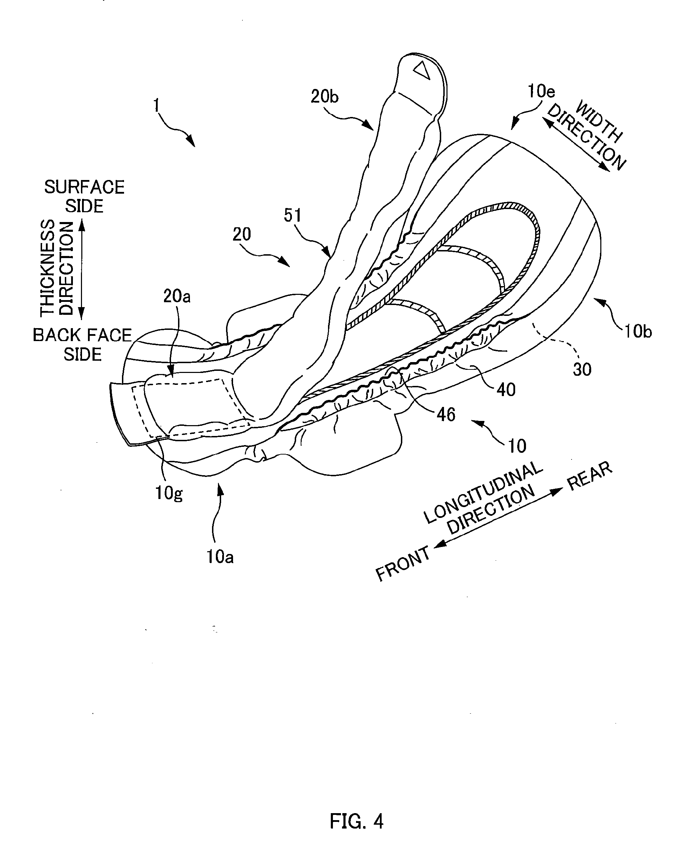

FIG. 1 is a plan view of a surface side of a base absorbent body 10; FIG. 2 is a plan view of a surface side of a top absorbent body 20; FIG. 3 is a diagram showing a state in which the base absorbent body 10 and the top absorbent body 20 are joined; FIG. 4 is a perspective view of a sanitary napkin 1. In the plan view of the base absorbent body 10 in FIG. 1, an external shape line of the top absorbent body 20 is virtually indicated by a dotted line. The sanitary napkin 1 (absorbent article) is a sanitary napkin having a two-layer structure and including the base absorbent body 10 (corresponding to the base body) and the top absorbent body 20 (corresponding to the absorbent body) that is placed on a surface of the base absorbent body 10.

In the sanitary napkin 1, as shown in FIG. 3, a point Z that is supposed to face the vaginal orifice (corresponding to the vaginal-orifice opposing section) is positioned on a center line CL of the sanitary napkin 1 in the width direction and on a side closer to the front with respect to the center in the longitudinal direction. In other words, in the sanitary napkin 1, a length of a back side with respect to the point Z that is supposed to face the vaginal orifice is formed longer than a length of a front side with respect to the point Z.

While a front end section 20a of the top absorbent body 20 is joined to a front end section 10a of the base absorbent body 10, a rear end section 20b of the top absorbent body 20 is a free end that can separate from the base absorbent body 10 using the front end section 20a as a fulcrum and can move. Therefore, a user of the sanitary napkin 1 first affixes the base absorbent body 10 to an inside face of an undergarment in such a manner as the longitudinal direction is along to a fore-and-aft direction of user's body; the user pulls up the rear end section 20b of the top absorbent body 20 with the undergarment being worn, and wears the sanitary napkin 1 in such a manner as to place the top absorbent body 20 into a recess such as buttocks. Fluid such as excreted menstrual blood is absorbed mainly by the top absorbent body 20.

A width of the middle part in the longitudinal direction (a narrow region 51, to be described in details later) of the top absorbent body 20 according to the present embodiment is narrow in the width direction compared with the front end section 20a and the rear end section 20b, as shown in FIG. 2.

Therefore, a user is easy to place the top absorbent body 20 into a recess such as buttocks, and the top absorbent body 20 is likely to be in close contact with the recess. Accordingly, it is possible to prevent fluid from leaking.

The base absorbent body 10 includes a fluid-permeable surface sheet, a fluid-impermeable back face sheet, and an absorbent-body base material (corresponding to the liquid-absorbent core) that is between the surface sheet and the back face sheet. On a surface sheet side of the base absorbent body 10, a pair of compressed recesses 15c, 15d (corresponding to the compressed section) are formed in which the surface sheet and the absorbent-body base material are compressed by a groove-embossing process in the thickness direction, and that extend along the longitudinal direction on both side sections of the base absorbent body 10 in the width direction, as shown in FIG. 1.

The sanitary napkin 1 according to the present embodiment is **characterized in that**, in a portion that is in the base absorbent body 10 and on which the narrow region 51 of the top absorbent body 20 is placed, a length W3 between the pair of compressed recesses 15c, 15d along the width direction is equal to or more than a length W1 of the narrow region 51 along the width direction.

Hereinbelow, constituent elements of the sanitary napkin 1 are each described in detail.

### < Base Absorbent Body 10 >

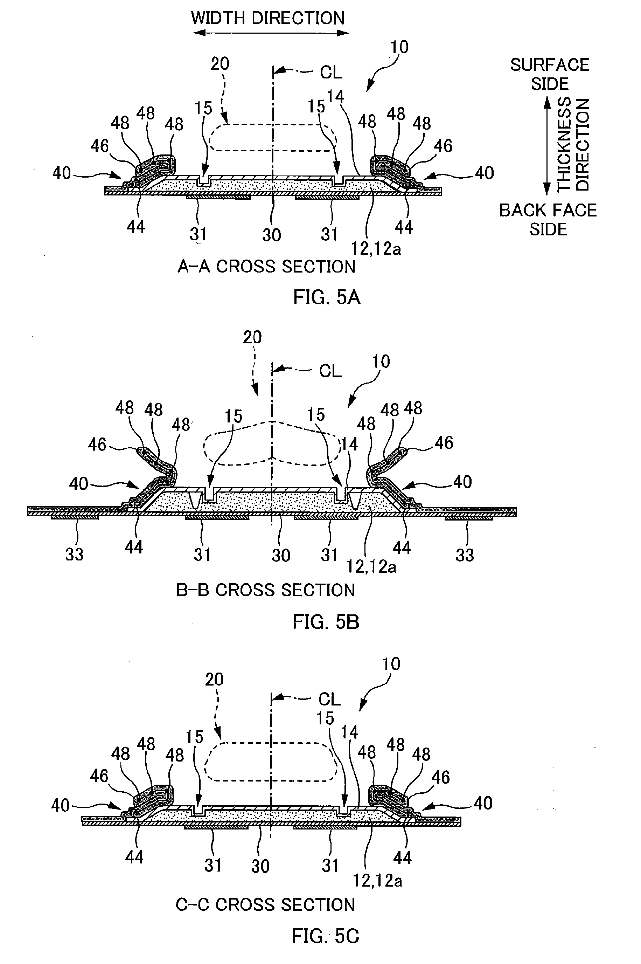

FIGS. 5A to 5C are respectively a cross-sectional view taken along A-A of FIG. 1, a cross-sectional view taken along line B-B of FIG. 1, and a cross-sectional view taken along line C-C of FIG. 1. In FIG. 5, only an external shape line of the top absorbent body 20 is virtually indicated by a dotted line.

A shape of the base absorbent body 10 as viewed from above is a substantial rectangle that is long in the longitudinal direction. The base absorbent body 10 includes: an absorbent-body base material 12 that absorbs fluid; a surface sheet 14 that is provided covering at least a surface side of the absorbent-body base material 12 throughout; a back face sheet 30 for preventing fluid absorbed in the absorbent-body base material 12 from leaking to a back face side; and a side sheet 40 for forming a leakage-proof wall 46 that prevents fluid from leaking outside in the width direction.

The absorbent-body base material 12 includes a pulverized-pulp layered body 12a obtained by layering pulverized pulp that is obtained by pulverizing pulp, superabsorbent polymer that is mixed into the pulverized-pulp layered body 12a, a fluid-permeable sheet such as tissue paper (not shown) that covers the pulverized-pulp layered body 12a.

The surface sheet 14 is a fluid-permeable sheet and its material is an appropriate nonwoven fabric; for example, air-through nonwoven fabric and spunlacing nonwoven fabric that are formed of synthetic resin fiber such as polyester and polyethylene undergo a hydrophilic treatment etc. and are used.

The surface sheet 14 and the absorbent-body base material 12 are compressed in the thickness direction with having hot-melt adhesive therebetween by the groove-embossing process, so that the surface sheet 14 and the absorbent-body base material 12 have been joined and integrated. The groove-embossing process is performed by a pair of sandwich-pressing members (not shown) opposing each other. One of the pair of sandwich-pressing members has a continuous rib-shaped protrusion. In addition, on a peak section of the rib-shaped protrusion, island-shaped protrusions are provided at intervals in a direction in which the rib-shaped protrusion is continuous. Besides, in the other sandwich-pressing member, a face opposing the rib-shaped protrusion is formed flat. Accordingly, as shown in FIG. 1, in the compressed recesses 15 that are formed on the surface sheet 14 and the absorbent-body base material 12 after the groove-embossing process, a low-compressed section 15a compressed at a low compression rate and a high-compressed section 15b compressed at a higher compression rate than that rate are formed alternately in a direction in which compressed recess 15 extends.

In a region in which this compressed recess 15 is formed, rigidity increases. When sandwiching between legs gives to the sanitary napkin 1 compression force in the width direction, the compressed recesses 15c, 15d formed on the both side sections in the width direction works as a starting point of folding, and a region between the pair of compressed recesses (a region between 15c and 15d) bulges towards user's body. On the other hand, a region located on side ends in the width direction with respect to the pair of compressed recesses 15c, 15d (sides close to the side sheet 40) folds along thighs. That is, even when the base absorbent body 10 is sandwiched between legs, the pair of compressed recesses 15c, 15d can prevent the base absorbent body from bending on the region between the pair of compressed recesses. Sandwiching between legs causes the region surrounded by the compressed recesses 15 to bulge in this manner, so that the top absorbent body 20 placed on the top of the base absorbent body 10 becomes easier to be supported from below. Also, it becomes easier to keep the top absorbent body 20 and the base absorbent body 10 in contact with each other, so that fluid absorbed by the top absorbent body 20 becomes more likely to forward to the base absorbent body 10.

The back face sheet 30 (corresponding to the fluid-impermeable sheet) is a fluid-impermeable sheet made of polyethylene, polypropylene, or the like, for example. A shape of the back face sheet 30 is longer than the absorbent-body base material 12 in the longitudinal direction, and wider than the absorbent-body base material 12 in the width direction. The absorbent-body base material 12 adheres to a surface side of the back face sheet 30 with hot-melt adhesive. On a front end section 30a and a rear end section 30b, the back face sheet 30 and the surface sheet 14 are joined by welding etc. (see the hatched areas in FIG. 1), so that the absorbent-body base material 12 is held between the back face sheet 30 and the surface sheet 14. Preferably, hot-melt adhesive is applied between layers of the sheets.

On the back face side of the back face sheet 30, an "anti-displacement affixing section 31" is provided that is for affixing the sanitary napkin 1 to the inside of an undergarment after placing the sanitary napkin 1 inside the undergarment, in order not to displace the sanitary napkin 1 from a position where the napkin has initially been placed. The anti-displacement affixing section 31 is, for example, hot-melt adhesive applied to a certain range in the back face of the back face sheet 30, and is formed continuing from the front end section 10a to a rear end section 10b of the base absorbent body 10.

Besides, in order to more securely prevent displacement of the undergarment and the sanitary napkin 1, wing sections 32 are respectively formed extending outside in the width direction on both ends of the back face sheet 30 in the width direction. "Anti-displacement affixing sections 33" are also provided on a back face of each wing section 32. The wing section 32 is folded outward, and is affixed to the outside of the undergarment by the anti-displacement affixing section 33. Note that, in the longitudinal direction, a position of the center between the wing sections 32 matches with the above-mentioned point Z that is supposed to face the vaginal orifice.

The side sheet 40 is for forming the leakage-proof wall 46 in the vicinity of both end sections of the absorbent-body base material 12 in the width direction, as shown in FIG. 5B. As shown in FIG. 1, the side sheet 40 is provided along longitudinal direction, covering the surface sheet 14 from a surface side. The side sheet 40 is made of a hydrophobic sheet; as its material, a spun-bonded nonwoven fabric that is formed of synthetic resin fiber such as polypropylene or polyethylene and the like are used, for example.

More specifically, as shown in FIG. 1, a pair of the side sheets 40 in the width direction are symmetrically disposed relative to the center line CL in the width direction, and extends along the longitudinal direction from the front end section 10a to the rear end section 10b, that is, to outer edges of the sanitary napkin 1. As shown in FIG. 5B, the side sheets 40 are each compression-bonded and fixed onto the surface sheet 14 in the vicinity of both end sections of the absorbent-body base material 12 in the width direction, and fixed sections 44 are formed. An end section 46 extending beyond this fixed section 44 is a free end. An elastic member 48 is fixed to this end section 46 along the longitudinal direction with stretching. Accordingly, when curving the sanitary napkin 1 in such a manner as the surface sheet 14 is inside, the elastic member 48 contracts, so that the end section 46 as the free end stands from the surface sheet 14 to be the leakage-proof wall 46. As shown in FIGS. 5A and 5C, a front end section and a rear end section, in the longitudinal direction, of the end section 46 serving as the leakage-proof wall are joined to the side sheet 40 with hot-melt adhesive lying toward the surface sheet 14, and are non-standing section that does not stand.

These side sheets 40 each extend further outward in the width direction from their respective fixed sections 44; more specifically, their respective outer edges reach outer edges of the back face sheet 30, that is, the outer edges of the sanitary napkin 1. The outer edges (see the hatched areas in FIG. 1) are joined with hot-melt adhesive, etc. to the outer edges of the back face sheet 30 in the width direction along the longitudinal direction throughout.

### < Top Absorbent Body 20 >

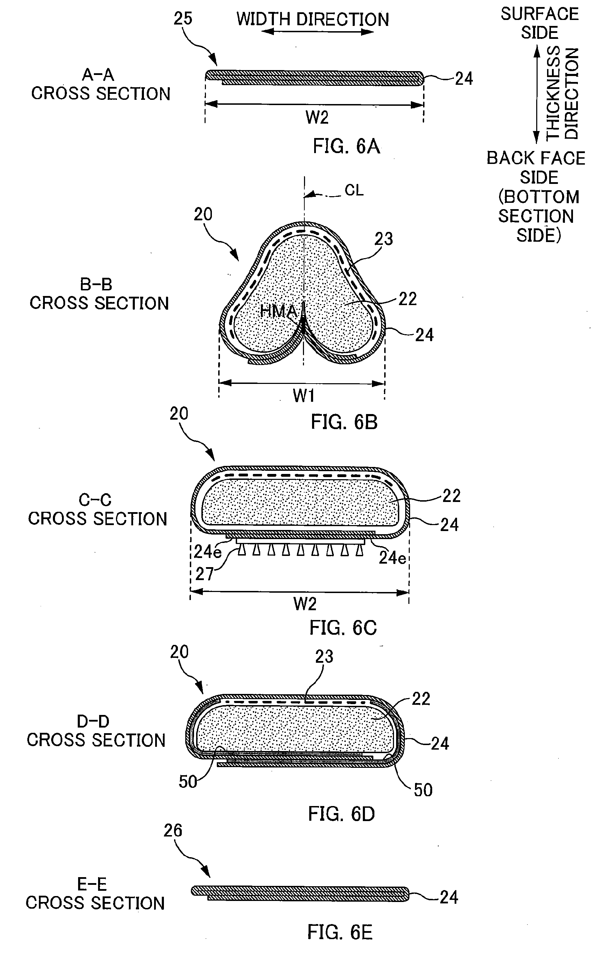

FIGS. 6A to 6E are respectively a cross-sectional view taken along A-A of FIG. 2, a cross-sectional view taken along line B-B of FIG. 2, a cross-sectional view taken along line C-C of FIG. 2, a cross-sectional view taken along line D-D of FIG. 2, and a cross-sectional view taken along line E-E of FIG. 2. The top absorbent body 20 includes a pulverized-pulp layered body 22 (corresponding to the liquid-absorbent member and liquid-absorbent core) that absorbs fluid, an intermediate sheet 23 that is disposed closer to a surface side compared with the pulverized-pulp layered body 22, and a shape-keeping sheet 24 (corresponding to the sheet) that wraps the pulverized-pulp layered body 22 and the intermediate sheet 23 together and keeps these pulverized-pulp layered body 22 and the like in a long shape elongated in the longitudinal direction. Further, as shown in FIG. 6B, in the middle part of the top absorbent body 20 in the longitudinal direction, a part that is in the shape-keeping sheet 24 and on the back face side where the sheet does not contact user's body (corresponding to the portion on the side where the sheet does not contact user's body, that is, the non-skin-contacting face) and another part that is in the shape-keeping sheet 24 and on the back face side are joined at the center in the width direction, in such a manner as to oppose each other along the longitudinal direction. As a result, the middle part of the top absorbent body 20 in the longitudinal direction serves as the narrow region 51, in which a width in the width direction is narrow compared with the front end section 20a and the rear end section 20b. A joined section 52 that joins the part and the other part of the shape-keeping sheet 24 is not exposed to a surface side, of the shape-keeping sheet 24, where the sheet contacts user's body (corresponding to the portion on the side where the sheet contacts the user's body, that is, the skin-contacting face). In FIG. 3, in order to show a position of the joined section 52, the joined section 52 is virtually indicated by dotted lines.

The intermediate sheet 23 is a fluid-permeable sheet having more excellent properties for drawing fluid than the shape-keeping sheet 24; as its material, an air-through nonwoven fabric that is formed of synthetic resin fiber such as polypropylene and the like are used, for example. The intermediate sheet 23 and the shape-keeping sheet 24 superpose and undergo a perforating embossing process. Thereby, the intermediate sheet 23 is joined and integrated into the shape-keeping sheet 24. However, the intermediate sheet 23 is not essential; for example, the shape-keeping sheet 24 may have the density gradient by the shape-keeping sheet 24 having a two layer structure.

The perforating embossing process is performed with a pair of devices (not shown) opposing each other. More specifically, conical protrusions are provided on one of the devices, and hole sections into which the protrusions are each inserted is provided on the other opposing one. The device on which the protrusion is formed is heated. When the conical protrusions penetrate the superposing intermediate sheet 23 and shape-keeping sheet 24 to form a large number of perforations 28 (see FIG. 2), edges of the perforations are heat-welded. Thereby, the intermediate sheet 23 is joined and integrated into the shape-keeping sheet 24.

The shape-keeping sheet 24 is a fluid-permeable sheet; as its material, the same material as the above-mentioned surface sheet 14 of the base absorbent body 10 is used, for example. The shape-keeping sheet 24 when being opened flat into a sheet has a substantially rectangular shape as view from above. Applying hot-melt adhesive to an entire surface of the shape-keeping sheet 24 opened flat, the shape-keeping sheet 24 is rolled into a cylinder as shown in FIG. 6C. Both end sections 24e in the width direction superposes and are joined with hot-melt adhesive, and the pulverized-pulp layered body 22 and the intermediate sheet 23 are accommodated in the cylinder throughout the longitudinal direction.

A front section and a rear section of the shape-keeping sheet 24 shown in FIG. 2 in the longitudinal direction are each folded without having the pulverized-pulp layered body 22 and the intermediate sheet 23, as shown in FIGS. 6A and 6E. Besides, hot-melt adhesive (not shown) is applied to the folded section, and compression-bonding by an embossing process is performed. Thereby, the front end section and rear end section of the shape-keeping sheet 24 in the longitudinal direction are sealed, to become thin sealed sections 25, 26. The rear sealed section 26 can work as a handle used when pulling up the top absorbent body 20.

Further, as shown in FIGS. 2 and 6D, in a portion that is in a rear area of the top absorbent body 20 and in which at least pulverized pulp exists, a leakage-proof sheet 50 is provided covering the pulverized-pulp layered body 22 from the back face side. The leakage-proof sheet 50 is a fluid-impermeable sheet, and has a function to prevent fluid absorbed by the pulverized-pulp layered body 22 from leaking outside the top absorbent body 20. More specifically, when using the sanitary napkin 1, there are cases in which the rear end section 20b of the top absorbent body 20 projects backward beyond a rear edge 10e of the base absorbent body 10. In this case, even if fluid penetrates onto an outer surface of (particularly, the back face) of the rear end section 20b of the top absorbent body 20, it is possible to prevent an undergarment from being soiled when the rear end section 20b of the top absorbent body 20 is brought into contact with the undergarment. As a material of the leakage-proof sheet 50, non-perforated film that completely interrupts fluid and is made of polyethylene, polypropylene, or the like is preferable. However, it does not have to completely interrupt fluid; for example, nonwoven fabrics made of water-repellent fiber and the like is also acceptable.

On the back face side of the top absorbent body 20, a fastening section 27 (FIG. 2) is provided that is for fixing the top absorbent body 20 onto a surface of the base absorbent body 10. This fastening section 27 can keep the top absorbent body 20 at an appropriate position that is adjusted when wearing the absorbent article. For the fastening section 27, an adhesive member, a hook member (a male member of hook-and-loop fasteners) and the like can be used. For example, regarding a hook member on which a plurality of needles are arranged that are tilted a certain degree from a plane, there is such a hook member that, when pulling in one direction, the hook member is unlikely to be hooked to fibers and is likely to be unfastened, and when pulling in another direction, the hook member is likely to be hooked to fibers and is likely to be fastened. Further, in order to make it easier to fasten, a female member of hook-and-loop fasteners may be provided on a side close to the base absorbent body 10, the female member corresponding to a male member of hook-and-loop fasteners provided on a side close to the top absorbent body 20.

### < Joining of Top Absorbent Body 20 and Base Absorbent Body 10 >

The top absorbent body 20 is placed on the surface side of the base absorbent body 10 with aligning their respective center line CL in a same position in the width direction. The top absorbent body and the base absorbent body are joined with placing the front end section 20a of the top absorbent body and the front end section 10a of the base absorbent body in a same position. As shown in FIG. 3, the sealed section 26 on a rear end side of the top absorbent body 20 projects backward beyond outer edges of the base absorbent body 10 to the extent that a user can pick up the sealed section. The sealed section 26 functions as a handle.

The front end section 20a of the top absorbent body 20 is permanently joined to the front end section 10a of the base absorbent body 10 firmly. In the present embodiment, hot-melt adhesive HMA (indicated as HMA in the drawings) is applied to a permanently-joined section 10g (the dark colored region in FIG. 1) of the base absorbent body 10; the region to which adhesive is applied adheres to the front end section 20a of the top absorbent body 20 so that the front end section 20a and the front end section 10a are permanently joined. Here, the permanently joining means inseparable state in which the top absorbent body 20 and the base absorbent body 10 are joined firmly to an extent that it is inevitable to occur breakage of at least either one of the top absorbent body 20 and the base absorbent body 10 when separating them deliberately. As other method of the permanently joining, there is the groove-embossing process, and the like.

### < Narrow Region 51 of Top Absorbent Body 20 and Compressed Recess 15 >

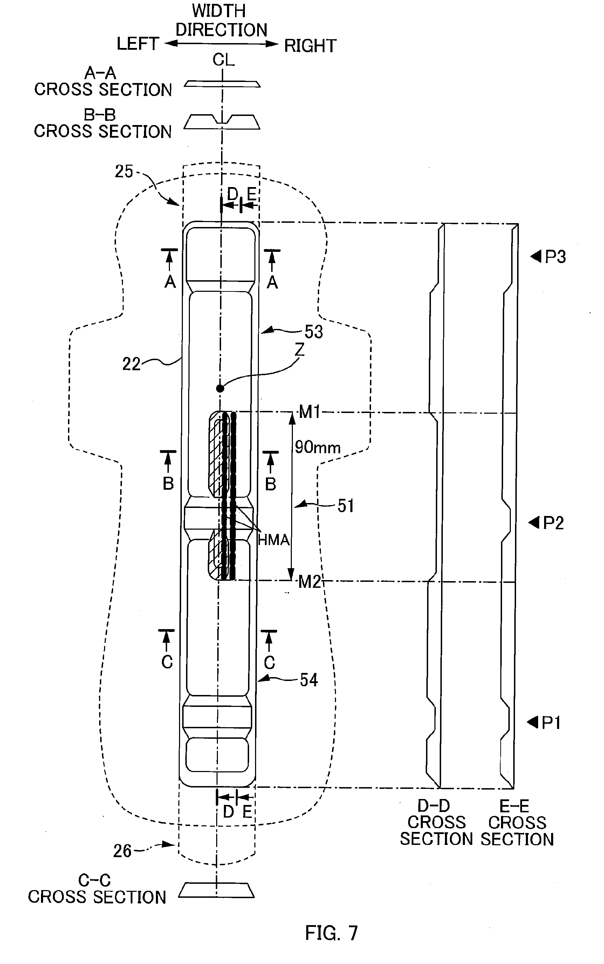

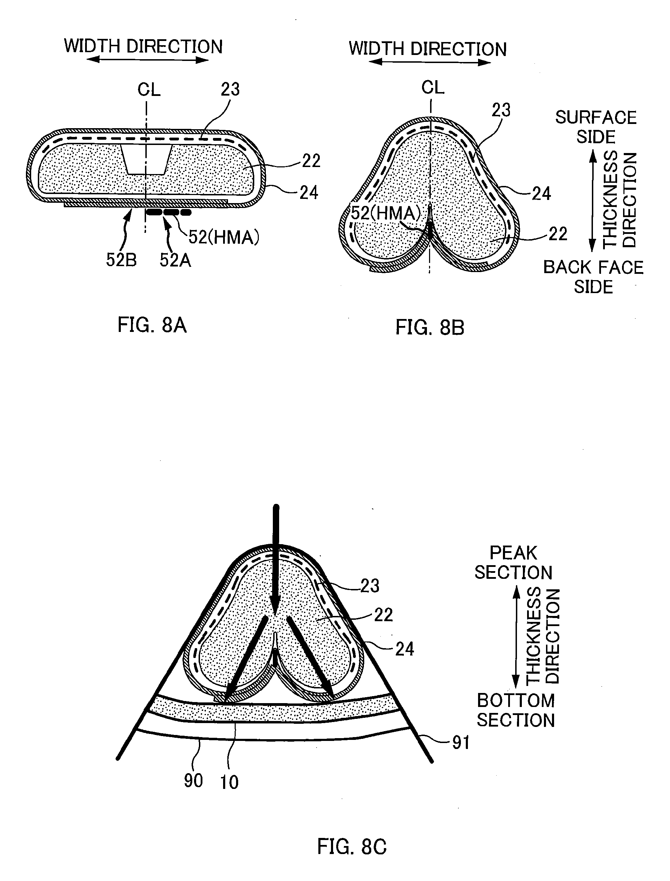

FIG. 7 is a diagram showing the distribution of basis weight of pulverized pulp 22 in the top absorbent body 20. FIG. 8A is a cross-sectional view of the top absorbent body before the narrow region 51 is formed; FIG. 8B is a cross-sectional view after the narrow region 51 is formed; FIG. 8C is a cross-sectional view how the narrow region 51 abuts a recess 91 of user's body (between the back of a vaginal orifice and an anus).

In the present embodiment, a region that is in the top absorbent body 20 and that abuts the recess 91 of user's body serves as the narrow region 51 whose width in the width direction is narrow compared with the front end section 20a and the rear end section 20b. That is, as shown in FIG. 2, the narrow region 51 whose length along the width direction is W1 (corresponding to the certain width) is positioned between a first region 53 whose length (W2) in the width direction is longer than the length W1 (certain width W1) of the narrow region 51 in the width direction and a second region 54 whose length (W2) in the width direction is longer than the length W1 (certain width W1) of the narrow region 51 in the width direction. The length W1 of the narrow region 51 in the width direction is uniform throughout an entire region in the longitudinal direction. The narrow region 51 corresponds to the minimum section in which a length of the top absorbent body 20 in the width direction is shortest in the middle part of the top absorbent body 20 in the longitudinal direction.

As shown in FIG. 7, in order to form the narrow region 51, hot-melt adhesive HMA is applied to a region that is in the top absorbent body 20 and that abuts the recess 91 of user's body, that is, behind the point Z that is supposed to face the vaginal orifice. More specifically, the hot-melt adhesive HMA is applied to an area from position M1 that is behind the point Z that is supposed to face the vaginal orifice to position M2, whose length is about a length of the recess 91 of user's body (for example, 90mm).

As shown in FIGS. 8A and 8B, a part 52A of the shape-keeping sheet 24 on the back face side and another part 52B of the shape-keeping sheet 24 on the back face side are joined with hot-melt adhesive HMA at the center in the width direction, so that the narrow region 51 is formed. Therefore, a region that is in the top absorbent body 20 and to which hot-melt adhesive HMA is applied serves as the joined section 52 that joins the part 52A and the other part 52B of the shape-keeping sheet 24.

Further, in the present embodiment, hot-melt adhesive HMA is applied to the middle part of the top absorbent body 20 in the longitudinal direction, continuously from the position M1 to the position M2. The part 52A and the other part 52B of the shape-keeping sheet 24 on the back face side are joined in such a manner as to oppose each other along the longitudinal direction throughout an area from the position M1 to the position M2. Therefore, a portion of the top absorbent body 20 from the position M1 to the position M2 serves as the narrow region 51. As shown in FIG. 3, in the vicinity of the narrow region 51, a length along the width direction is slightly short compared with the front end section 20a and the rear end section 20b of the top absorbent body, due to the influence of the narrow region 51.

If the narrow region 51 is not provided in the top absorbent body 20, when a user attempts to cause the top absorbent body 20 to be in close contact with the recess 91, it is impossible to place the top absorbent body 20 into the recess 91 as quickly as in the present embodiment. Particularly, in a case of a user who is thin and flesh such as buttocks is not well-developed, it is difficult to sandwich between his/her own flesh the top absorbent body 20 that is not narrow.

Therefore, as the top absorbent body 20 according to the present embodiment, the narrow region 51 is provided in the region that abuts the recess 91 of user's body. This makes it easier to place the top absorbent body 20 into the recess 91 of user's body, and also makes it easier for the recess 91 to be in close contact with the top absorbent body 20. As a result, fluid leakage is prevented.

Further, in the present embodiment, this top absorbent body 20 that is easy to be in close contact with user's body is realized by joining the part 52A and the other part 52B of the shape-keeping sheet 24 on the back face side; a manufacturing method of the top absorbent body is comparatively easy. Therefore, it is possible to inexpensively mass-produce the sanitary napkin 1 according to the present embodiment.

In the top absorbent body 20 according to the present embodiment, the parts of the shape-keeping sheet 24 on the back face side are joined with hot-melt adhesive HMA in order not to expose the joined section 52 forming the narrow region 51 to a side where the shape-keeping sheet 24 is in contact with user's body (surface side). Therefore, it is possible to prevent hot-melt adhesive HMA that solidifies to be hard or is sticky from contacting user's skin, and also possible to prevent a user from feeling discomfort. Besides, since chemicals such as hot-melt adhesive HMA do not contact user's skin, it can be said that the sanitary napkin according to the present embodiment is hygienic sanitary napkin.

In other words, in the present embodiment, the parts, on the back face side, of the shape-keeping sheet 24 containing the pulverized pulp 22 are joined; so that easy manufacturing of sanitary napkins that is likely to be in close contact with the recess 91 of user's body are realized. Besides, since the joined section is not exposed to a side where the napkin contacts user's body, it is possible to manufacture a sanitary napkin that is comfortable to wear.

Further, as shown in FIGS. 7 and 8A, in a region that is in the top absorbent body 20 and corresponds to the narrow region 51, a basis weight of the pulverized pulp 22 in the center in the width direction is less than a basis weight of the pulverized pulp 22 in the both side sections in the width direction (B-B cross section, D-D cross section) in order to easily join the parts of the shape-keeping sheet 24 on the back face side. The center in the width direction, in which an amount of the pulverized pulp 22 is small, works as a starting point of folding; so that it is likely to form a right-left symmetrical peak as shown in FIG. 6B. As a result thereof, the narrow region 51 reaches a bottom of the recess of user's body. In contrast, in the first region 53 positioned ahead of the narrow region 51 and the second region 54 positioned behind the narrow region 51, there is not a case where the pulverized pulp 22 in the center in the width direction is particularly less than the pulverized pulp 22 in the both side sections in the width direction. In other words, weights (g/m²) of the first region 53 and the second region 54 (C-C cross section) are more than a weight of the narrow region 51. As mentioned above, reducing the weight of the narrow region 51 minimizes the consumption of the pulverized pulp 22.

The first region 53 and the second region 54 are comparatively flat (FIG. 6C), compared with the narrow region 51 (FIG. 6B) in which the parts of the shape-keeping sheet 24 on the back face side are joined. Since the second region 54 positioned behind the narrow region 51 abuts user's waist (back), it is possible to prevent a user from feeling uncomfortable (from feeling a gap) when the user is lying in order to sleep or the like. Excreted fluid is likely to flow to the waist through the recess 91 when the user is lying. A length of the second region 54 that abuts the waist of user's body is comparatively wide in the width direction; so that fluid leakage can be prevented and an appearance of the absorbent article gives the user a sense of security. Besides, the second region 54 having the fastening section 27 provided thereon is flat (FIG. 2), so that it is possible to surely fasten the top absorbent body 20 to the base absorbent body 10 when wearing the absorbent article.

In a similar manner, the first region 53 ahead of the narrow region 51 is flat, and the top absorbent body 20 and the base absorbent body 10 are permanently joined with the front end sections 10a, 20a surely. Further, since the narrow region 51 is behind the point Z that is supposed to face the vaginal orifice in the present embodiment, it is possible to receive the excreted fluid with the absorbent body whose area is comparatively wide; as a result thereof, fluid leakage is unlikely to occur.

Regarding user's body, as mentioned above, a perineum at the back of a vaginal orifice is a recess (concave section), but an area in the vicinity of the vaginal orifice is a comparatively projection section because a labium majus has developed there. Thus, as the present embodiment, it is preferable that, around the vaginal orifice (projection section), the top absorbent body 20 deforms into a concave section in such a manner to cover a labium majus (the flat first region 53 curves), and that, in a perineum (recess) at the back of the vaginal orifice, the top absorbent body 20 has a peak (FIG. 8C) in such a manner to be placed into the recess. As the present embodiment, the parts, on the back face side, of the shape-keeping sheet 24 of the top absorbent body 20 are joined to form the narrow region 51 (the top absorbent body 20 are folded to make a peak), so that a boundary between the area around the vaginal orifice and perineum, the top absorbent body 20 deforms smoothly (from a concave section to a peak). Therefore, it is possible to cause the top absorbent body 20 to be in close contact with user's body throughout the fore-and-aft direction (longitudinal direction), and fluid leakage is unlikely to occur.

In the top absorbent body 20 according to the present embodiment, when fluid flowing from a peak section of the recess 91 reaches the joined section 52 of the narrow region 51, the fluid divides into right and left in the width direction and moves to the base absorbent body 10. Therefore, it is possible to absorb fluid with the entire top absorbent body 20, and to utilize the top absorbent body 20 effectively.

In FIG. 7, hot-melt adhesive HMA is applied to around the center line CL only, and is not applied throughout an area from the center line CL to a right end of the top absorbent body 20. This is because, if hot-melt adhesive HMA is applied to areas near both ends (left and right ends) of the top absorbent body 20 in the width direction, there is a risk that the top absorbent body 20 and the base absorbent body 10 adhere to each other when wrapping. However, a position for applying the hot-melt adhesive HMA is not limited thereto. The hot-melt adhesive HMA may be applied to the areas near the both ends of the top absorbent body 20 in the width direction. This enables the narrower narrow region 51 to be formed, so that the top absorbent body 20 becomes likely to be placed into the bottom of the recess of user's body. As hot-melt adhesive HMA to be used, adhesive such as olefin-based one that has low tackiness (low stickiness) when being solidified is preferable.

Further, hot-melt adhesive HMA is not applied throughout an area from the center line CL to the both ends (left and right ends) in the width direction, so that, in a cross-sectional shape of the narrow region 51, a length along the width direction becomes longer from a peak section to a bottom section, as shown in FIG. 8C. This allows the top absorbent body 20 to be in close contact with the recess 91 of user's body along from the peak section of the recess 91 to side faces of the recess 91.

In FIG. 7, hot-melt adhesive HMA is applied to only a right region with respect to the center line CL, but the invention is not limited thereto. The hot-melt adhesive HMA may be applied to both of right and left regions between which the center line CL is sandwiched. However, if the top absorbent body 20 is folded using the center line CL as a folding position in such a manner to make a peak along the center line CL, applying hot-melt adhesive HMA to a region only on either one side (right side or left side) in the width direction with respect to the center line CL can reduce consumption of adhesive.

Incidentally, in the sanitary napkin 1 according to the present embodiment, the top absorbent body 20 and the base absorbent body 10 can separate in the other regions than the permanently-joined section 10g shown in FIG. 1. Therefore, when a user wears the napkin, the user places the top absorbent body 20 into a recess of user's body, and adjusts a position of the top absorbent body 20. For the top absorbent body 20 whose position has been adjusted, the fastening section 27 of the top absorbent body 20 is fastened to the base absorbent body 10 (or undergarment). As a result, the top absorbent body 20 is positioned with being placed into the recess of user's body. When wearing the absorbent article, the region between the pair of compressed recesses (the region between 15c and 15d) in the base absorbent body 10 curves toward user's body by sandwiching between user's legs. Accordingly, the top absorbent body 20 placed into the recess of user's body can be supported by the region between the pair of compressed recesses 15c, 15d. Here, among regions between the pair of compressed recesses 15c, 15d on the base absorbent body, the region on which the narrow region 51 of the top absorbent body 20 is placed is defined as a "curved section".

Even if the top absorbent body 20 is placed into the bottom of the recess so that the curved section and the top absorbent body 20 are not in contact with each other, the curved section can be positioned comparatively close to the top absorbent body 20 placed into the recess. Therefore, even if the top absorbent body 20 is gradually removed from the bottom of the recess when being worn, the curved section can support the top absorbent body 20 from below. Accordingly, it is possible to prevent the top absorbent body 20 from being removed completely from the recess.

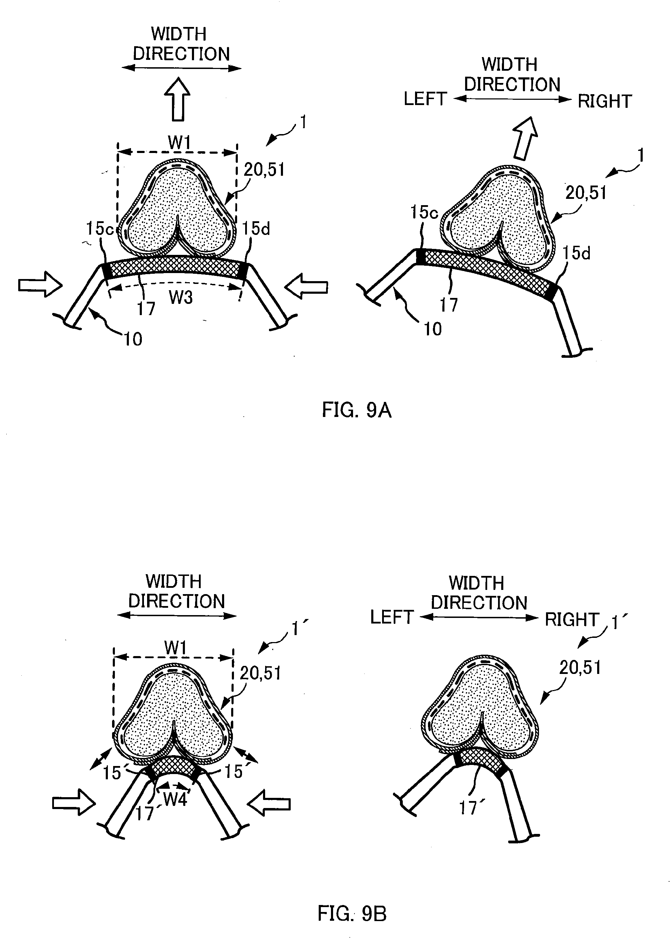

FIG. 9A is a cross-sectional view of the narrow region 51 and a curved section 17 when the sanitary napkin 1 of the present embodiment is worn, and FIG. 9B is a cross-sectional view of the narrow region 51 and a curved section 17' when a sanitary napkin 1' according to a comparative example is worn. In the sanitary napkin 1' according to the comparative example, a length W4 of the curved section 17' (a region that is in the base absorbent body 10' and between the pair of compressed recesses 15' extending in the longitudinal direction) in the width direction is shorter than the length W1 of the top absorbent body 20 in the width direction in the narrow region 51. With this sanitary napkin 1', an area of the curved section 17' is smaller than an area of a bottom face of the narrow region 51, so that it is impossible to support in a stable manner the top absorbent body 20 placed into the recess of user's body. Accordingly, the top absorbent body 20 is likely to be removed from the recess of the body in the user's hard movement.

Thus, in the sanitary napkin 1 according to the present embodiment, the length W3 of the curved section 17 in the width direction is longer than the length W1 of the narrow region 51 in the width direction (note that since the compressed recesses 15c, 15d each have a certain width in the width direction, the length W3 of the curved section 17 in the width direction is a length from a left end position of the compressed recess 15c to a right end position of the compressed recess 15d). Therefore, the area of the curved section 17 is larger than the area of the bottom face of the narrow region 51, so that it is possible to support in a stable manner the top absorbent body 20 place into the recess of user's body. Accordingly, it is possible to keep the top absorbent body 20 in close contact with the recess.

Further, for example, in the sanitary napkin 1' according to the comparative example, when a user tilts to the right in the width direction, the curved section 17' of the base absorbent body 10' that is affixed to an undergarment follows user's movement and tilts to the right. However, there is a possibility that the curved section 17', whose area is small, cannot cause the top absorbent body 20 to tilt to the right. Accordingly, the top absorbent body 20 becomes likely to be removed from the recess. In contrast, in the sanitary napkin 1 according to the present embodiment, the curved section 17, whose area is large, can cause the top absorbent body 20 to tilt to the right. Accordingly, it is possible to keep the top absorbent body 20 in close contact with the recess.

In other words, in the sanitary napkin 1 according to the present embodiment, in the region that is in the base absorbent body 10 and on which the narrow region 51 is placed, a length of the region (curved section 17) between the pair of compressed recesses along the width direction is equal to or more than a length of the narrow region 51 in the width direction. Therefore, the sanitary napkin 1 can support in a stable manner by the curved section 17 the top absorbent body 20 placed into the recess of user's body, and is a sanitary napkin that is likely to be in close contact with user's body.

### < Wrapping of Sanitary Napkin 1 >

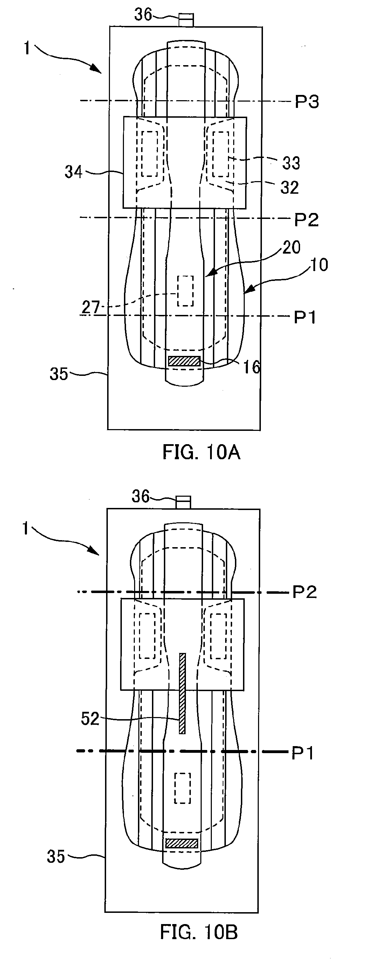

FIG. 10A is a diagram showing how the sanitary napkin is wrapped individually. The top absorbent body 20 and the base absorbent body 10 are joined and folded in four at three folding positions P1, P2, P3 in the longitudinal direction. Then, the top absorbent body 20 and the base absorbent body 10 are wrapped together by one sheet of wrapping sheet 35, and tape 36 is attached thereto.

As shown in FIG. 7, three folding positions P1, P2, P3 along the width direction in the longitudinal direction, a basis weight of the pulverized pulp 22 is less throughout the width direction than in portions that are adjacent to each of the folding positions P1, P2, P3 in the longitudinal direction, so that the sanitary napkin 1 is likely to be folded in four (the base absorbent body 10 is the same as mentioned above).

As shown in FIG. 10A, the wing section 32 is folded toward the top absorbent body 20 and wrapped. The wing section 32 and a protection sheet 34 adhere to each other in such a manner as adhesive 33 applied onto a back face of the wing section 32 opposes a face of the protection sheet 34, to which a parting agent is applied. Thereby, when a user unwraps the sanitary napkin 1, adhesive 33 transfers to and remains on the wing section 32 due to a function of the parting agent, so that an "anti-displacement affixing section 33" is formed on the wing section 32. By sandwiching the top absorbent body 20 between the protection sheet 34 and the base absorbent body 10 in this manner, it is possible to prevent the top absorbent body 20 from shifting when the top absorbent body 20 is folded. In a similar manner, the fastening section 27 functions to prevent the top absorbent body 20 from shifting when the top absorbent body 20 is folded.

Further, when individual wrapping, in the rear end section (temporarily joined section 16), the top absorbent body 20 and the base absorbent body 10 may be temporarily joined by the embossing process and the like separably. Temporarily joining at the rear end section 16 makes it possible to prevent the top absorbent body 20 from shifting relative to the base absorbent body 10, and to prevent the top absorbent body 20 from being wrapped with being bent.

### < Modified Example of Wrapping of Sanitary Napkin 1 >

FIG. 10B is a diagram showing a wrapping form in which the sanitary napkin 1 is folded in three. In this modified example, the top absorbent body 20 and the base absorbent body 10 that are joined and folded in three at two folding positions P1, P2 in the longitudinal direction, and are wrapped together by one sheet of wrapping sheet 35.

At this stage, it is necessary for hot-melt adhesive HMA (joined section 52) not to exist on the folding positions P1, P2 along the width direction. This makes it possible to avoid folding the napkin on the narrow region 51 in which the parts of the shape-keeping sheet 24 on the back face side are joined. Therefore, the joined section 52 is difficult to separate and is easy to be folded.

### === Second Embodiment ===

In the foregoing embodiment, the top absorbent body 20 and the base absorbent body 10 are permanently joined inseparably on the permanently-joined section 10g. However, in the second embodiment, the top absorbent body 20 and the base absorbent body 10 are separable on a portion corresponding to the permanently-joined section 10g (hereinafter referred to as a joined portion).

For example, while a female member of hook-and-loop fasteners is fixed to the joined portion of the base absorbent body 10, a male member of the hook-and-loop fasteners is fixed, corresponding to the female member, on the back face side of the front end section 20a of the top absorbent body 20. This makes the top absorbent body 20 and the base absorbent body 10 detachable at the joined portion.

Therefore, it is possible to wrap separately the base absorbent body 10 and the top absorbent body 20 (not shown). More specifically, only the base absorbent body 10 is placed on a wrapping sheet, and then the base absorbent body 10 is folded in four together with the wrapping sheet at certain folding positions P1, P2, P3 and wrapped. On the other hand, regarding the top absorbent body 20, when only the top absorbent body 20 is placed on another wrapping sheet, the top absorbent body 20 is folded in four together with the wrapping sheet at certain folding positions P1, P2, P3 and wrapped. Preferably, the base absorbent body 10 and the top absorbent body 20 have the same folding positions. Having the same folding positions makes joining easier because their respective folding positions can superpose when joining the top absorbent body 20 and the base absorbent body 10.

If the base absorbent body 10 and the top absorbent body 20 are wrapped separately in this manner, a certain number of wrapped units of the base absorbent body 10 and a certain number of wrapped units of the top absorbent body 20 are each grouped, and are accommodated into their respective containers, and two units are prepared. Further, these two units are accommodated into a single package, and this package is supplied to the market as a minimum sales unit. However, the minimum sales unit is not limited thereto; for example, a package of the top absorbent body 20 and a package of the base absorbent body 10 can be sold separately.

If the top absorbent body 20 and the base absorbent body 10 are sold separately, there are the following advantages. For example, it is not necessary to have different types of sanitary napkins such as ones for day use and ones for overnight use, so that reserves of sanitary napkins at home can be reduced. As a sanitary napkin for day use in which an amount of menstrual blood is small, only the base absorbent body 10 is used. On the other hand, when a napkin is used for overnight use in which an amount of menstrual blood is large, the base absorbent body 10 to which the top absorbent body 20 is joined is used. In other words, a sanitary napkin for day use can be modified and used for overnight use, so that reserves at home can be reduced. In addition, when the top absorbent body 20 is used during a certain period of time and has absorbed menstrual blood sufficiently, it is possible to replace the top absorbent body 20 only without replacing the base absorbent body 10. This makes it possible to reduce waste since frequency of replacement of the base absorbent body 10 is reduced.

Incidentally, if the top absorbent body 20 and the base absorbent body 10 are wrapped separately, a nonwoven fabric such as an SMS nonwoven fabric (a nonwoven fabric having a three-layer structure in which a melted-blown nonwoven fabric is sandwiched between top and bottom spun-bonded nonwoven fabrics) is preferable as a material of a wrapping sheet for the top absorbent body 20, for example. Adhesive for anti-displacement is not applied to the back face side of the top absorbent body 20, unlike the base absorbent body 10. Therefore, if a wrapping sheet is a nonwoven fabric, the top absorbent body 20 can be effectively fastened in an inter-fiber empty space of the wrapping sheet by a male member of hook-and-loop fasteners for joining or the fastening section 27. Accordingly, it is possible to prevent displacement of the top absorbent body 20 in width direction when being folded in four. This makes it possible to give a good impression of an appearance on a user when unwrapping.

### === Third Embodiment ===

In the foregoing embodiments, in a portion that is in the base absorbent body 10 and on which the narrow region 51 of the top absorbent body 20 is placed, the length W3 between the pair of compressed recesses 15c, 15d along the width direction is equal to or more than the length W1 of the narrow region 51 along the width direction. However, the invention is not limited thereto. The length between the pair of compressed recesses 15c, 15d along the width direction may be shorter than the length of the narrow region 51 along the width direction. The top absorbent body 20 according to the third embodiment, as shown in FIGS. 8A and 8B, is a top absorbent body 20 (absorbent body): that includes the pulverized-pulp layered body 22 (corresponding to the liquid-absorbent core) absorbing fluid; and the shape-keeping sheet 24 (corresponding to the sheet) that wraps the pulverized-pulp layered body 22, and that has the skin-contacting face positioned on a side where the shape-keeping sheet 24 contacts user's body, and the non-skin-contacting face positioned on a side where the shape-keeping sheet 24 does not contact user's body; the top absorbent body 20 having a joined section where the part 52A of the shape-keeping sheet 24 on the non-skin-contacting face (back face side) and the other part 52B of the shape-keeping sheet 24 on the non-skin-contacting face (back face side) are joined in such a manner as the part 52A and the other part 52B of the shape-keeping sheet 24 oppose on the center in the width direction along the longitudinal direction. A region that is in the top absorbent body 20 and to which hot-melt adhesive HMA is applied corresponds to the joined section 52 that joins the part 52A and the other part 52B of the shape-keeping sheet 24.

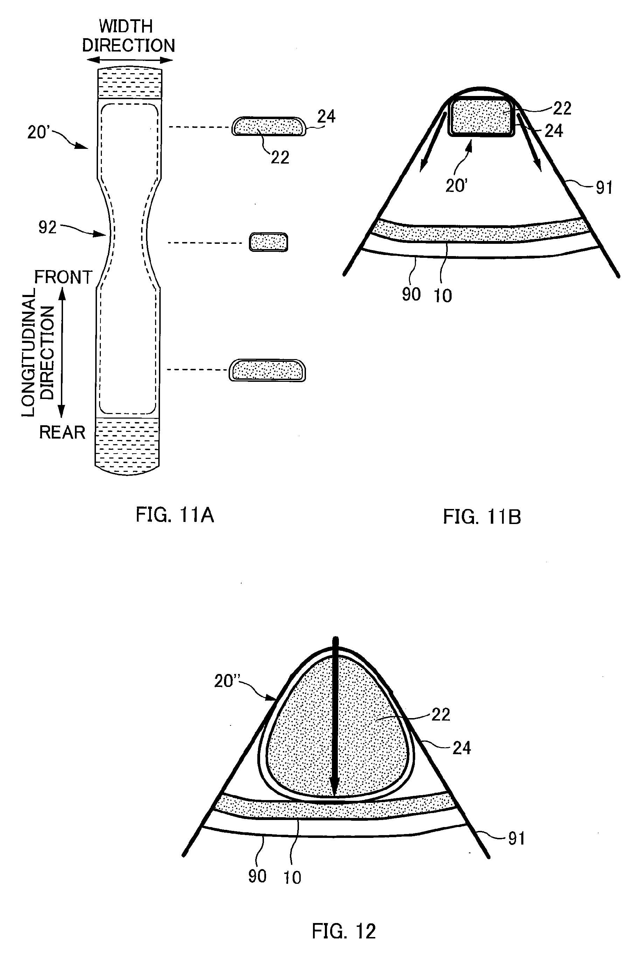

Hereafter, compare the top absorbent body 20 according to the third embodiment and a top absorbent body according to the comparative example, which is different from the top absorbent body 20 according to the third embodiment. FIG. 11A is a plan view and a cross-sectional view of the top absorbent body 20' according to the first comparative example, and FIG. 11B is a cross-sectional view showing how the top absorbent body 20' according to the first comparative example abuts the recess 91 of user's body. In the top absorbent body 20' of the first comparative example, a basis weight of the pulverized pulp 22 in a region abutting the recess 91 is less than a basis weight in other regions and a constriction 92 is formed in order to cause the top absorbent body 20' to be in close contact with the recess 91 of user's body. A length of this constriction 92 along the width direction is shorter than a length of the other regions along the width direction; that is, the constriction 92 is narrower than the other regions.

In this case, in a similar manner to the top absorbent body 20 of the third embodiment, the top absorbent body 20' of the first comparative example is likely to be placed into the recess 91, and the top absorbent body 20' can abut the peak section of the recess 91. However, while the top absorbent body 20 of the third embodiment is in close contact with an entire region of the recess 91 (peak section and side faces) (FIG. 8C), the top absorbent body 20' of the first comparative example cannot contact the side faces of the recess 91, as shown in FIG. 11B. Therefore, there is a risk that fluid leaks from between the recess 91 and the top absorbent body 20' through the side face of the recess 91.

Further, in a constriction 92 according to the first comparative example, a basis weight of a pulverized pulp 22 for absorbing fluid is small compared with the narrow region 51 of the top absorbent body 20 according to the third embodiment. In other words, for the absorption capacity of the top absorbent body that abuts the recess 91 of user's body, the first comparative example (constriction 92) is smaller than the third embodiment (narrow region). Accordingly, if a large amount of fluid is excreted or fluid is repeatedly excreted, the top absorbent body 20' of first comparative example cannot absorb the fluid, and there is a risk that fluid overflows and leakage is caused.

In the top absorbent body 20 of the third embodiment, that a region that abuts the recess 91 has a height (thickness) in the thickness direction is realized. In contrast, in the top absorbent body 20' of the first comparative example, a region that abuts the recess 91 is narrow, so that a height in the thickness direction is insufficient compared with the third embodiment. Therefore, in the top absorbent body 20 of the third embodiment, even if the narrow region 51 is placed into the recess 91 as shown in FIG. 8C, the base absorbent body 10 and the narrow region 51 can be in contact with each other, so that fluid absorbed with the narrow region 51 is easy to move to the base absorbent body 10. Accordingly, it is possible to prevent a user from feeling discomfort (wet and sticky).

FIG. 12 is a diagram showing how the top absorbent body 20" according to the second comparative example abuts the recess 91 of user's body. The top absorbent body 20" of the second comparative example has a triangle cross section, and can be in close contact with the entire region of the recess 91 (peak section and side faces). However, in the top absorbent body 20 of the third embodiment, when fluid from the peak section of the recess 91 reaches the joined section 52 of the narrow region 51, the fluid divides into right and left in the width direction and moves to the base absorbent body 10. In contrast, in the top absorbent body 20" of the second comparative example, fluid moves in one direction: moving downward in the thickness direction (vertical direction) from the peak section of the recess 91, or moving through high-density region in which the pulverized pulp 22 is gathered. Therefore, there is a risk that only a part of regions of the top absorbent body 20" absorbs fluid. That is, in the top absorbent body 20 of the third embodiment, in which the fluid divides and moves into two direction, it is possible to absorb the fluid with the entire top absorbent body 20, and to effectively use the top absorbent body 20, compared with the top absorbent body 20" of the second comparative example.

When the pulverized pulp 22 is used as a material for absorbing fluid as the third embodiment, it is difficult to shape the top absorbent body 20" in such a manner as to have a triangle cross section.

Further, a common sanitary napkin is long in the longitudinal direction, so it is generally folded and wrapped individually. Therefore, when being folded, its triangle shape deforms and triangle cross section cannot be maintained, so that the top absorbent body 20" cannot be in close contact with the recess 91 of user's body. In similar manner, if the sanitary napkin 1 according to the third embodiment is folded for individual wrapping, it is difficult to maintain a peak-shaped cross-section of the top absorbent body 20 as shown in FIG. 6B. However, in the present embodiment, parts, on the back face side, of the shape-keeping sheet 24 of the top absorbent body 20 are joined, to form the narrow region 51. Therefore, sandwiching the top absorbent body 20 between the recess allows the top absorbent body 20 to be in close contact with the entire recess 91, as shown in FIG. 8C.

Regarding user's body, as mentioned above, a perineum at the back of a vaginal orifice is a recess (concave section), but an area in the vicinity of the vaginal orifice is a comparatively projection section because a labium majus has developed there. Thus, as the third embodiment, it is preferable that, around the vaginal orifice (projection section), the top absorbent body 20 deforms into a concave section in such a manner to cover a labium majus (the flat first region 53 curves), and that, in a perineum (recess) at the back of the vaginal orifice, the top absorbent body 20 has a peak (FIG. 8C) in such a manner to be placed into the recess. As the third embodiment, the parts, on the back face side, of the shape-keeping sheet 24 of the top absorbent body 20 are joined to form the narrow region 51 (the top absorbent body 20 are folded to make a peak), so that a boundary between the area around the vaginal orifice and perineum, the top absorbent body 20 deforms smoothly (from a concave section to a peak). Therefore, it is possible to cause the top absorbent body 20 to be in close contact with user's body throughout the fore-and-aft direction (longitudinal direction), and fluid leakage is unlikely to occur.

### === Other Embodiments ===

In the foregoing embodiments, the rear end sections of the top absorbent body 20 and the base absorbent body 10 (temporarily joined section 16 in FIG. 10) are separable (possible to split). However, these rear end sections may be permanently joined inseparably in a similar manner to the front end sections. Besides, the top absorbent body 20 and the base absorbent body 10 may be permanently joined inseparably throughout the entire region in the longitudinal direction. However, if the narrow region 51 of the top absorbent body 20 can separate from the base absorbent body 10, this makes it easier to place the top absorbent body 20 into the recess of user's body. In addition thereto, this allows the top absorbent body 20 to be in close contact with the recess of user's body.

In the foregoing embodiments, the oval compressed recess 15 is formed on the base absorbent body 10, but this invention is not limited thereto. For example, it is acceptable that, without enclosing the center of the base absorbent body 10 by the oval compressed recess, the pair of compressed recesses extends along the longitudinal direction at the both side sections of the base absorbent body 10 in the width direction. In the compressed recess 15 according to the foregoing embodiment, the low-compressed section 15a and the high-compressed section 15b are lined up alternately, as shown in FIG. 1, and the surface sheet 14 and the absorbent-body base material 12 are continuously compressed along the longitudinal direction; but the invention is not limited thereto. For example, dot-like compressed sections formed by the embossing process may be formed lined up discontinuously in the longitudinal direction.

Besides, in the region that is in the base absorbent body 10 and on which the narrow region 51 is placed, three or more compressed recesses may be formed along the longitudinal direction. In this case, if a length, in the width direction, of the base absorbent body 10 between a compressed recess that is located closest to one side (right side) in the width direction and a compressed recess that is located closest to another side (left side) is equal to or more than the length of the narrow region 51 in the width direction, it is possible to keep the top absorbent body 20 be in close contact with the body. This is because the base absorbent body 10 is likely to be folded at the compressed recesses closest to side ends in the width direction as a starting point of folding, and the top absorbent body 20 is supported by a region between the pair of compressed recesses closest to side ends.

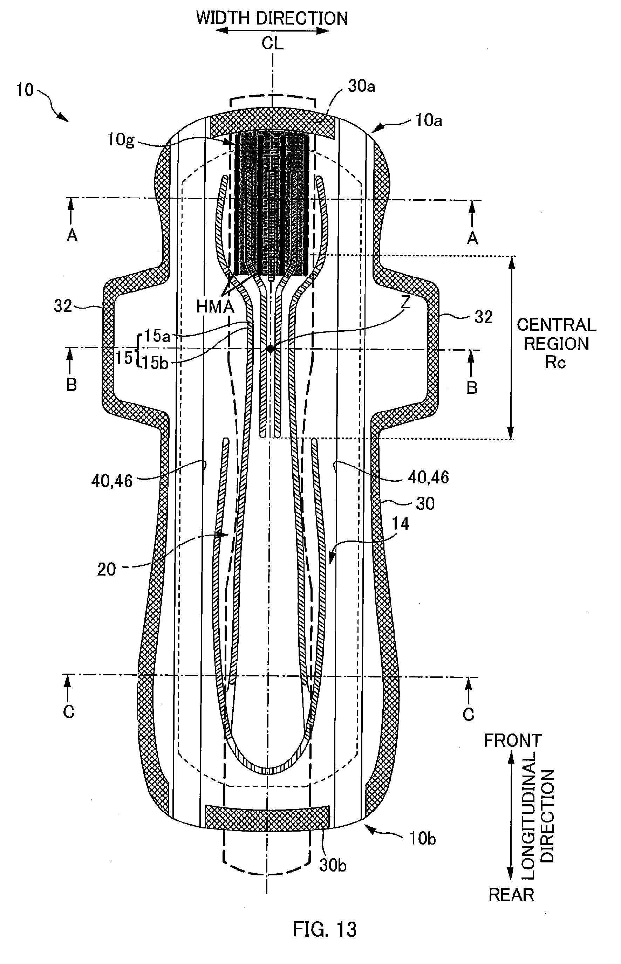

FIG. 13 is a plan view of the surface side of the base absorbent body 10 that is different from the foregoing embodiments. In the region in which this compressed recess 15 is formed, rigidity increases. As shown in FIG. 13, since about four compressed recesses are formed on the base absorbent body 10 along the longitudinal direction, the base absorbent body 10 is unlikely to deform in the width direction. For example, a central region Rc where the wing sections 32 (to be described later) extending in the width direction exist abuts a groin of user's body and is sandwiched from the width direction. Therefore, if rigidity of the central region Rc is small in the width direction, the base absorbent body 10 cannot withstand pressure by being sandwiched between a groin and is folded along the center line CL. As a result thereof, the top absorbent body 20 is sandwiched from both sides between the base absorbent body 10, so that creases, etc. made along the longitudinal direction on the top absorbent body 20 makes it more difficult for the top absorbent body 20 to be in close contact with a recess of buttocks. The compressed recess 15 is formed selectively onto a region that needs the above-mentioned rigidity.

In the foregoing embodiments, by joining the parts of the shape-keeping sheet 24 on the back face side, the length of the middle part (the narrow region 51) of the top absorbent body 20 in the longitudinal direction is shortened in the width direction than those of the front end section 20a and the rear end section 20b of the top absorbent body 20. However, the invention is not limited thereto. For example, it is also acceptable that the basis weight of the pulverized pulp 22 in the middle part of the top absorbent body 20 in the longitudinal direction is less than basis weights in other regions, to form the constriction. When, in the region that is on the base absorbent body and on which the constriction is placed, a length along the width direction between the pair of compressed recesses is equal to or more than a length of the constriction in the width direction, the constriction can be in close contact with a recess of user's body in a stable manner. However, since the constriction is narrow as a whole compared with the narrow region 51 of the present embodiment, the constriction cannot be in contact with the side faces of the recess of the body. As a result thereof, fluid is likely to leak from between the recess and the constriction. In addition, since the basis weight of pulverized pulp in the constriction is small compared with the narrow region 51, there is a risk that fluid overflows when a large amount of fluid is excreted. However, on the other hand, compared with the foregoing embodiments, it is not necessary that, in the top absorbent body having the constriction, adhesive is applied to the back face side of the shape-keeping sheet 24 and the parts of the shape-keeping sheet 24 are joined. Therefore, this makes a manufacturing method even easier.

In the foregoing embodiments, the absorbent article is provided to a user with the part 52A and the other part 52B on the back face of the shape-keeping sheet 24 being joined by the joined section (a region to which hot-melt adhesive HMA is applied). However, the invention is not limited thereto. For example, though the top absorbent body 20 has the joined section, the absorbent article may be provided to a user with the part 52A and the other part 52B of the shape-keeping sheet 24 not being joined. For example, it is also acceptable that a user joins the part 52A and the other part 52B on the back face of the shape-keeping sheet 24 after unwrapped by the user, by applying the adhesive (joined section) to the back face side of the top absorbent body 20 and providing a protection sheet between the wrapping sheet and the adhesive. In this case, user's task increases compared with the foregoing embodiments. However, in contrast to the joined top absorbent body, the top absorbent body is easy to be folded and there is no risk that the joined section separates because the flat top absorbent body 20 is folded in individual wrapping.

In the foregoing embodiments, the narrow region 51 is provided in the middle part of the top absorbent body 20, and the regions positioned ahead of or behind the narrow region 51 are wider than the narrow region 51. However, the invention is not limited thereto. If the joined section 52 at least exists in a region that is in the top absorbent body 20 and abuts a recess of user's body, it is possible for the top absorbent body 20 to be likely to be placed into the recess of user's body. Therefore, the joined section 52 is preferably located, in the longitudinal direction, between the point Z that is supposed to face the vaginal orifice of the top absorbent body 20 and the rear end section 20b (corresponding to the one end); the joined section 52 is more preferably positioned behind the point Z that is supposed to face the vaginal orifice as the foregoing embodiment. This allows fluid excreted from a vaginal orifice to be absorbed with a comparatively wide region, and fluid is difficult to leak.

In the foregoing embodiments, the base body (base absorbent body 10) includes an absorbent-body base material that is for absorbing fluid. However, if the base body includes a fluid-impermeable sheet, the base body does not have to include an absorbent-body base material. When the base body includes a fluid-impermeable sheet, it is possible to prevent fluid absorbed by the absorbent body (top absorbent body 20) from penetrating an undergarment.

The above embodiments are for the purpose of elucidating the understanding of the invention, and are not construed as limiting the invention in any way. The invention can be modified or improved without departing from the gist thereof, and any equivalents thereof are of course included in the scope of the invention.

## Claims

1. An absorbent body (20), comprising:
a liquid-absorbent core (22); and
a sheet (24)
that wraps the liquid-absorbent core, and
that has a skin-contacting face positioned on a side where the sheet contacts a user's body, and a non-skin-contacting face positioned on a side where the sheet does not contact the user's body;
the absorbent body (20) having a width direction and a longitudinal direction intersecting the width direction,
the absorbent body (20) having a joined section (52) where a part of the sheet on the non-skin-contacting face and another part of the sheet on the non-skin-contacting face are joined in such a manner as the part and the other part oppose on a center in the width direction along the longitudinal direction.

2. An absorbent body according to claim 1, wherein
the absorbent body (20) has a vaginal-orifice opposing section that opposes a vaginal orifice of the user's body,
one end of the absorbent body (20) in the longitudinal direction is located on a side close to buttocks of the user's body, and
the joined section is located between the vaginal-orifice opposing section and the one end, in the longitudinal direction.

3. An absorbent body according to claim 1 or 2, wherein
a narrow region (51) whose length along the width direction is a certain width is located between a first region (53) and a second region (54) in the longitudinal direction,
a length of the first region along (53) the width direction is longer than the certain width,
a length of the second region (54) along the width direction is longer than the certain width, and
the narrow region (51) is a region in which the part and the other part are joined by the joined section.

4. An absorbent body according to claim 3, wherein
a weight of the narrow region (51) is less than a weight of the first region (53) and a weight of the second region (54).

5. An absorbent body according to any of claims 1 to 4, wherein
the joined section does not exist on a folding position that is along the width direction and where the absorbent body (20) is folded.

6. An absorbent article, comprising:
a base body (10) having a fluid-impermeable sheet (30);
an absorbent body (20) according to any of claims 1 to 5 that is provided placed on the base body (10).
